# EUROPEAN PATENT SPECIFICATION

(11) **EP 1 206 258 B1**
(45) Date of publication and mention of the grant of the patent: **26.05.2004**
(21) Application number: 00907863.5
(22) Date of filing: 09.03.2000
(51) Int. Cl.: A61K 31/216, A61P 33/00

(54) **COMPOSITIONS CONTAINING ESTERS FOR TREATING PARASITIC INFESTATIONS OF ORGANISMS**
ESTER ENTHALTENDE ZUSAMMENSETZUNGEN ZUR BEHANDLUNG VON PARASITISCHEM BEFALL IN ORGANISMEN
COMPOSITIONS A TENEUR EN ESTERS DESTINEES AU TRAITEMENT DES INFESTATIONS PARASITAIRES D'ORGANISMES

(30) Priority: 24.08.1999 GB 9919889
(43) Date of publication of application: 22.05.2002
(73) Proprietor: LOVESGROVE RESEARCH LIMITED, Machynlleth, Powys SY20 8AX, Wales (GB)
(72) Inventor: GARNETT, David, John, Aberystwyth, Ceredigion SY23 3NY (GB)
(74) Representative: Lees, Kate Jane
(86) International application number: PCT/GB2000/000865
(87) International publication number: WO 2001/013913

(56) References cited:
- EP-A- 0 428 276
- US-A- 3 928 413
- US-A- 4 477 361
- AZIZ, N. H. ET AL: "Comparative antibacterial and antifungal effects of some phenolic compounds" MICROBIOS (1998), 93(374), 43-54 , XP000938201
- DATABASE WPI Section Ch, Week 199227 Derwent Publications Ltd., London, GB; Class C03, AN 1992-223400 XP002150518 & JP 04 149103 A (TAISHO PHARM CO LTD), 22 May 1992 (1992-05-22)

## Description

The present invention relates to compounds for the improved treatment of infestations, particularly but not exclusively infestations of crops, livestock and domestic animals such as stored grain, sheep and poultry.

A number of types of insects and mites are parasitic and may infect both domestic and farm animals causing serious health problems to the animal if left untreated. Some species burrow into the skin of the animal whilst other pierce the skin causing irritation and inflammation. These lesions are suscepticle to additional fungal or secondary insect infection. Thus there is a need for a treatment for insect and mite infestations that also provides anti-mycotic or mycostatic action. Insect and/or mite infestation and fungal contamination of crops, stored grains and food also has a serious adverse economic effect.

*Psoroptes ovis* is an ectoparasite of sheep causing psoroptic mange that causes inflammation and surface exudation, severe pruritus, wool loss, restlessness, biting and scratching of infested areas leading ultimately to death of the sheep in as little as 4-6 weeks. Another type of mite, namely *Varroa* infects beehives and afflicts *Apis mellifera* in many countries of the world.

Conventionally, organophosphates and synergised pyreithrins have been used to treat infestations. However, organophosphates have been implicated in the etiology of medical conditions in humans and must be handled with great care. Although synergised pyreithrins are generally considered to be a much safer alternative to the use of organophosphates, such compounds have been associated with deleterious effects on the environment, especially if allowed to enter the water system. ,

Animals, particularly sheep, may also suffer from blowfly strike *(ovine mysiasis).* Primary ovine strike is a cutaneous infestation by the larvae of green bottles *(Ovis aries)* of domestic sheep. The fly larvae infest and actively feed on the tissues and secretions of the animal. In temperate regions, such as Great Britain, the most common species responsible for this cutaneous infestation is the primary strike species *Lucilia sericata* Meigen (Diptera: Calliphoridae). Animals struck by the blowfly and left untreated may suffer from chronic ammonia toxicity leading ultimately to death. In England and Wales, 80% of farms are affected by this ectoparasite per annum, with 1.6% of all sheep being struck every year. Thus, this cutaneous infestation is a significant threat to livestock.

It is an aim of the present invention to provide compounds for an improved treatment against parasitic infestations which are safer to use and less damaging to the environment.

A further aim of the present invention is to provide compounds that not only provide a treatment against parasitic infection but also provide simultaneous treatment against secondary fungal infection.

Accordingly, the present invention provides the use of compounds for the manufacture of a medicament for the treatment of parasitic infestations of livestock, the compounds having the general formula:- 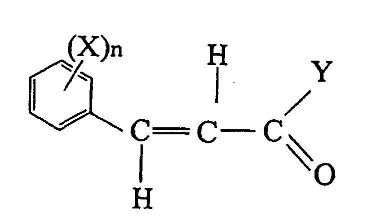 wherein Y is an alkyl group having 1 to 4 carbon atoms, a hydroxyl group, an amine group, a halide group or a nitro group; X is a hydroxyl group, an amine group, a halide group, a nitro group, an alkoxy group or an ester group and n is 0 or 1. In particular, the invention provides the use of the aforementioned compounds for the preparation of a medicament for the treatment of parasitic infestations selected from the group consisting *of Psoroptes sp., Varroa jacobsoni oudeman, Dermanyssus gallinae and Sarcoptes sp.*

The preferred compound is *trans*-cinnamic acid ethyl ester.

Additionally, the compounds have been found to be effective as a fungicide. This enables the compounds to be used to treat a combination of parasitic infestations in a diverse range of organisms such as poultry, sheep and bees and simultaneously reduce and/or prevent secondary fungal infection.

The compounds may be administered in a variety of forms depending upon the intended application of the compound. For example, the compound may be provided as a dilutable emulsion, being mixed with water and/or a suitable emulsifier such as sodium lauryl sulphate or lecithin. Such an emulsion may be used as a spray to treat organisms infested with, for example, mites or blowfly eggs, such as *Lucilia sericata*, or alternatively as a dip, particularly for the treatment of sheep. Preferably, a dip emulsion contains the active compound at a concentration of 0.1 to 10%.

Preferably, the concentrated formulation is at least 40 wt.% of the active compound and at least 40 wt.% water. More preferably, 50 wt.% of the formulation is made up of the active compound. Preferably, 1-5 wt.% of triton X-100 or powdered (de-oiled) lecithin is included in the formulation, more preferably 3 wt.%. If lecithin is used, it is preferable to use a chemically or enzymatically hydrolyzed type. Other compounds, such isopropyl alcohol or polyethylene glycol esters, may also be used in the formulation. The concentrated emulsion may then be diluted as appropriate for the intended application.

The formulation preferably includes minor amounts of an anti-oxidant, such as ascorbic acid or, more preferably, butylated hydroxytoluene (BHT) and/or preservatives, such as Nipagin, proprionic acid or Parabens.

Alternatively, solid formulations may be provided, for example for the treatment of stored grain or the like. The compound may be mixed with an inert carrier, such as silica talc.

The compound may alternatively be introduced into the area containing the infested organism by means of a wick based evaporator whereby the compound is vapourised in a sufficient concentration to kill the parasite but does not produce toxic effects in the infested organism. The compound may also be absorbed or adsorbed on material for slow release into the region of infestation. The compound may alternatively be included with an oily ointment or an aqueous cream for topical treatment of animals.

The active compounds may also be used in combination with other agents, such as allyl proprionate, to augment its activity.

The present invention will now be further illustrated by means of the following Examples in which Example 1 relates to the use of the preferred compound of the present invention for the control of the mite *Psoroptes cuniculi,* Example 2 relates to the use of the preferred compound of the present invention for the control of the mite *Acarus siro,* Example 3 relates to the use of the preferred compound of the present invention in the control of the mite *Varroa jacobsoni oudemans* Example 4 relates to the use of the preferred compound of the present invention in the control of *Lucilia sericata* infestation in sheep, and Example 5 relates to the antifungal activity of the preferred compound of the present invention, and with reference to the accompanying drawings in which:-
Figure 1 is a diagram illustrating the mean LT₅₀ for the mite *Psoroptes cuniculi* following their immersion in a range of concentrations of the compound *trans-* cinnamic acid ethyl ester;
Figure 2 is a diagram illustrating the proportion of *P. cuniculi* which were dead (± s.e.) 24 hours after immersion in a range of concentrations of *trans-*cinnamic acid ethyl ester;
Figure 3 is a diagram illustrating the mean LT₅₀ (± s.e) for the mite *P. cuniculi* for adult males (1), adult females (2) and female nymphs (3);
Figure 4 is a diagram illustrating efficacy of the compound trans-cinnamic acid ethyl ester against the eggs of the primary agent of sheep myiasis *Lucilia Sericata* after exposure for 1 minute expressed using the probit transformation;
Figure 5 is a diagram illustrating efficacy of the compound *trans-cinnamic* acid ethyl ester against the eggs of the primary agent of sheep myiasis *Lucilia Sericata* after exposure for 1 minute without using probit transformation;
Figure 6 is a diagram illustrating efficacy of the compound trans-cinnamic acid ethyl ester against the eggs of the primary agent of sheep myiasis *Lucilia Sericata* after exposure for 30 minutes; and

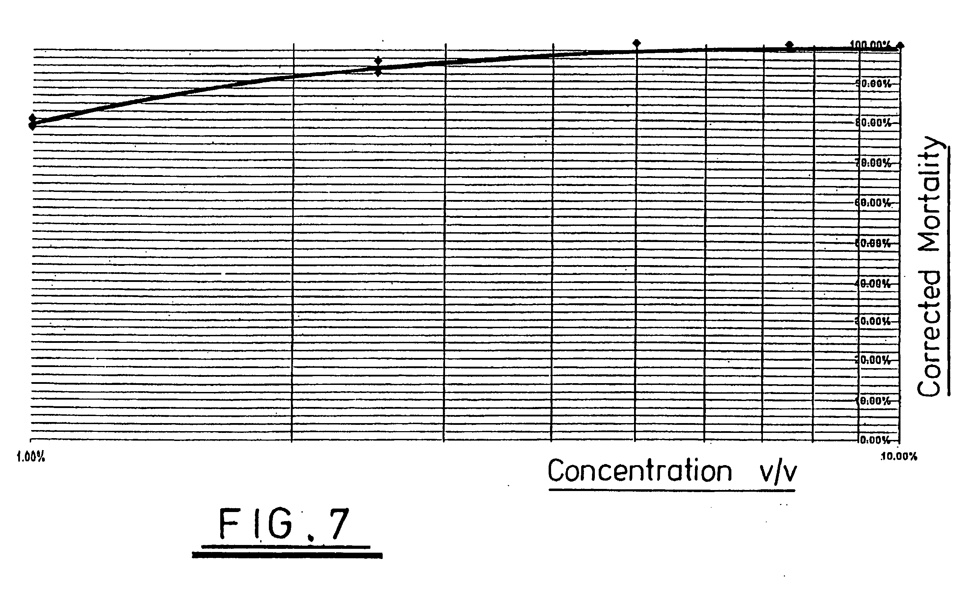

Figure 7 is a diagram illustrating efficacy of the compound *trans*-cinnamic acid ethyl ester against the eggs of the primary agent of sheep myiasis *Lucilia Sericata* after exposure for 90 minutes.

The present invention provides an effective treatment for a wide range of parasites, such as mites and the larvae of blow flies which cause detrimental effects to, inter alia, animals and insects. Additionally, the invention provides a treatment that also has antifungal activity.

The compound *trans*-cinnamic acid ethyl ester or ethyl cinnamate has been found to control infestation of mites in diverse organisms such as sheep, rabbits and bees. The compound is also effective against *L.sericata* eggs which cause a cutaneous infestation in sheep. Advantageously, the compound may be used on wounds and open lesions of animals without injury. This is unexpected since organic esters normally impede the healing of a lesion and/or produce toxic effects in the sheep. The compound may be provided in a variety of compositions depending upon the organism to be treated.

### Experimental. - Preparation of trans-Cinnamic Acid Ethyl Ester.

Trans-cinnamic acid ethyl ester is prepared in the conventional manner from the esterification of cinnamic acid with ethanol and a catalytic quantity of sulphuric acid, as illustrated in Scheme 1 shown below:-

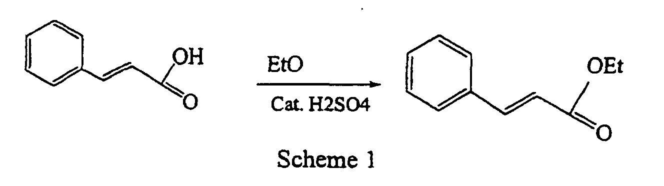

### Example 1.

A series *of in vitro* assays were conducted to examine the effects of the compound trans-cinnamic acid ethyl ester on the mite *Psoroptes cuniculi.* This mite infests rabbits and is very similar to the mite *Psoroptes ovis* which infests sheep and causes sheep scab.

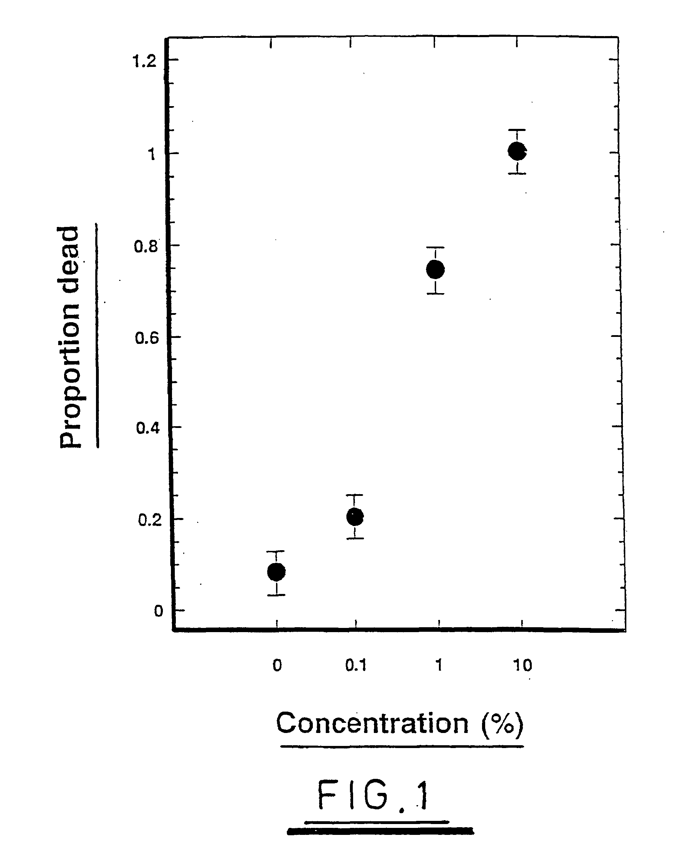

The mites were taken from the ears of infested rabbits and exposed for 24 hours to the active compound at concentrations of 10, 1 or 0.1 % (V/V). The concentrations were obtained by serial dilution of the compound in 0.05% sodium dodecyl sulphate (SDS). It was found that 100, 74 and 20% of the mites had died respectively compared to 8% following exposure to the control (0.05% SDS only), as illustrated in Figure 1 of the accompanying drawings.

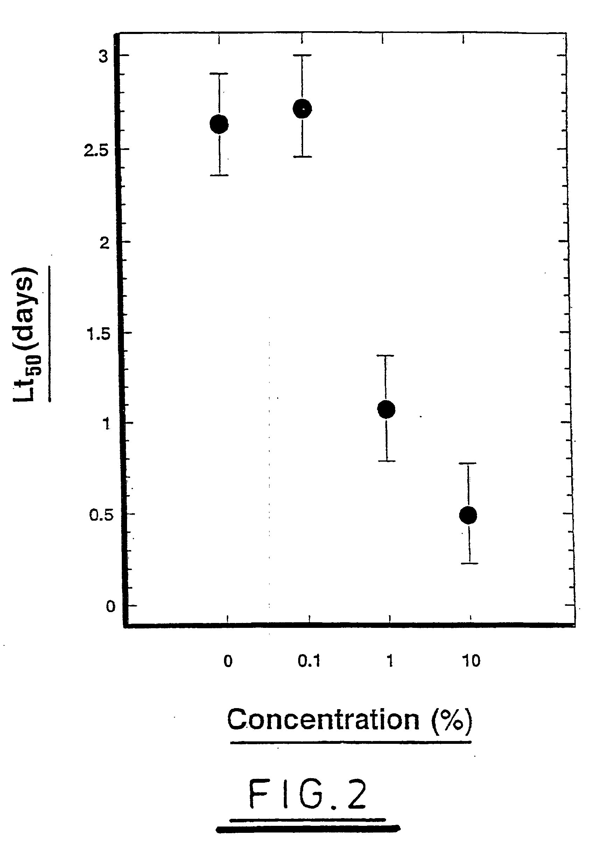

Exposure of the mites to concentrations of 10% and 1% of the ethyl ester resulted in 50% mortality (LT₅₀) after 0.5 days and 1 day respectively, compared to an LT₅₀ of approximately 2.5 days following exposure to 0.1% of the compound (V/V) or the control (0.05% SDS only). This is illustrated in Figure 2 of the accompanying drawings. Immersion of the mites in the compound for 1, 30 or 90 minutes showed no significant difference in either the LT₅₀ or the proportion dead 24 hours after the exposure thus demonstrating that exposure of the mite to the compound for one minute is as effective as exposure for 90 minutes.

The LC₅₀ (concentration required to produce 50% mortality) 24 hours after exposure to the ethyl ester was 2.21 % (95% confidence interval 1.73-2.92%), as shown in Table 1 below. The LC₉₅ was 6.29% (95% confidence interval 4.98-8.88).

**Table 1**

| **LD** | **Concentration** **(% V/V)** | **95% Confidence** **Interval** |
|---|---|---|
| 25 | 0.53 | -0.15 - 1.01 |
| 50 | 2.21 | 1.73 - 2.92 |
| 75 | 3.88 | 3.12 - 5.31 |
| 95 | 6.29 | 4.98 - 8.88 |

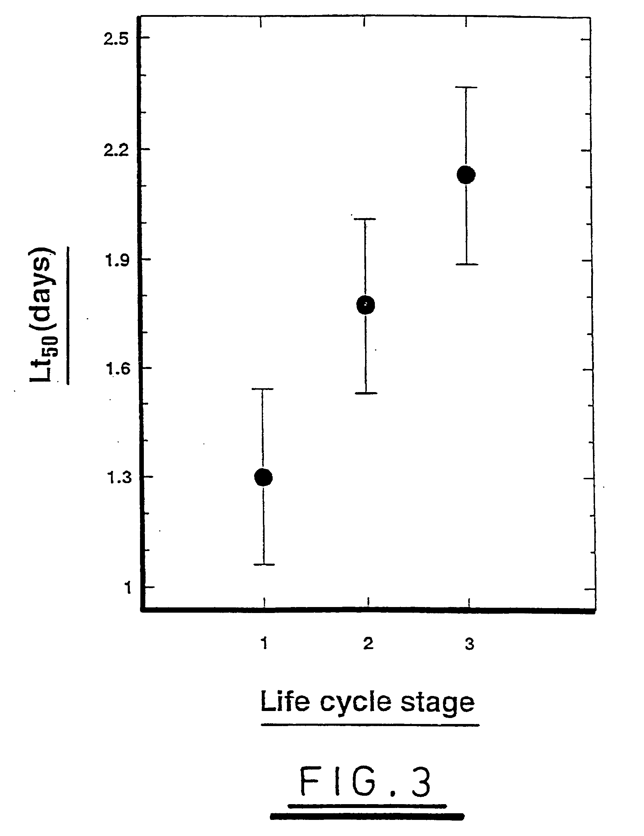

Figure 3 of the accompanying drawings shows the mean LT₅₀ (± s.e) for the mites at different stages of the life cycle. Line (1) represents adult males, line (2) adult females and (3) female nymphs. The mortality of the various life cycle stages of the mites was not significantly different in the first 24 hour exposure to the compound. However, the results indicated that beyond the first 24 hour period female nymphs had a lower mortality rate than adult males or females.

Thus, *trans-*cinnamic acid ethyl ester gives greater than 50% levels of mortality within 24 hours, for all life cycle stages of P. *cuniculi* after short contact times (1 min) at concentrations of 2.2% (V/V). At one minute contact time, concentrations of greater than 6% (V/V) are required to bring about mortality of greater than 95%. Recent theoretical analysis using simulation modelling (Wall, unpublished) has shown that sustained mortality of greater than 50% per day is required to suppress a growing population of *P. ovis.* Hence, the use of the compound in its present formulation as an effective control agent for *P. ovis* may require either use in relatively high concentrations (>6% V/V) or, if used at lower concentrations, on the presence continued residual activity on the host animal.

### Example 2.

The effect of *trans*-cinnamic acid ethyl ester on the mite *Acarus siro* was investigated using a residual filter paper bioassay. Seven samples of 0.01g of mite culture was placed on 7, 90mm Whatman™ filter papers. Each paper was previously treated with 745µl of a solution containing trans-cinnamic acid ethyl ester at concentrations varying from 74.64µl to 7.64 x 10⁻⁴ µl/petri dish respectively and allowed to dry for 15 minutes. The controls were treated in the same way except ethanol (HPLC grade) was used as this was sample dilutant. Percentage control was calculated as in W.S. Abbot, A Method of Computing the Effectiveness of an Insecticide, Journal of Economic Entomology (1925) 18, 265-267.

Table 2a below shows the efficacy of the compound against the mite *in vitro.* At a concentration of 7.64µl/petri dish, *trans*-cinnamic acid ethyl ester was found to be effective resulting in a percentage control figure of 100%.

**Table 2a**

| Concentration (µl) | Mean Percentage Mortality Over Control |
|---|---|
| 3.20 x 10¹ | 100.00 |
| 3.20 | 95.57 |
| 3.20 x 10⁻¹ | 97.74 |
| 3.20 x 10⁻² | 69.51 |
| 3.20 x 10⁻³ | 32.95 |
| 3.20 x 10⁻⁴ | 6.33 |

A further study was carried out to investigate the effect of Ethyl cinnamate on the grain/flour mite *Acarus siro* using a residual filter paper bioassay. Mites from a colony grown at 25°C ± 85% RH were removed using a spatular and placed into a petri dish. 275 µ of the cinnamate was placed onto a filter paper in one well of a 6 well (17 ml) cell culture plate and likewise, 275 µ of DH₂O was placed on another piece of 4 cm Whatman filter paper in a second well. Mixed instars (male + female) were added to each well. The actual number of instars was unknown in each case, since the test only sought to establish the presence of activity; measured in terms of cidal effect (knockdown). The duration of the test was 5 ± days. Both treatments were incubated at 25°C ± 85% RH and each well was checked 4 times daily. Small amounts of food mix of yeastea 20B and wheat germ in the ratio 3 : 1 were included on the filter paper in both treatments.

The treatment was later modified : 2, 10 cm Whatman filter papers were cut to ø of 3 cm and placed in the mite culture. After 10 minutes they were removed using forceps. The mites were then swept into each well respective of treatment. The cinnamate provided too glutenous (viscous) to be used undiluted. Therefore, it was dissolved using 10 fold dilutions in ethanol. Therefore 30 µ cinnamate : 270 µ ethanol to the extent of 10-₄. The control was ethanol.

The above experiment was repeated. The results are given in Table 2b below.

**Table 2b**

| **Concentration** | **% mortality** |
|---|---|
| Ethanol control | 24.00 |
| 10-₁ | 100.00 |
| 10-₂ | 100.00 |
| 10-₃ | 98.40 |
| 10-₄ | 86.00 |

The results above are very conclusive. Ethyl cinnamate is toxic to mites even at dilutions of 10-₄. It is now clear that *Acarus siro* can receive 100% mortality from an undiluted dose of Ethyl cinnamate in less than 15 minutes approximately.

Additionally, it was also noted that Ethyl cinnamate is toxic to insects, at least mealworms, T.molibr. However, the concentrations tested were 10-₁; 10-₂. 5 mealworms were placed onto each filter paper treatment. Although all were of the same instar, the 10-₁ individuals were slightly larger. They did not die and after two days moulted. 10-₇ did die. Therefore, the size of larvae appears to be important.

### Example 3.

The *trans*-cinnamic acid ethyl ester may also be used to control Varroa mite infections in bee hives caused by the *Varroa jacobsoni oudemans* mite which afflicts *Apis mellifera* in many countries of the world. The compound may be introduced into the hive using a simple wick based evaporator such as the Nassenheider Evaporator which produces a concentration of vapour in the hive which will kill the mites but not produce toxic effects on the bees. The compound is found naturally in Storax Oil and other plant material and therefore may be considered to be a safer treatment for control of the mites than the use of organophosphates or synergised pyreithrins.

The safety of the compound in man has also been determined using LD₅₀ assays using four animal species. Table 3 below illustrates the results of the assays.
Additionally, the ethyl ester compound produced no sensitization after a 24 hour closed-patch test on 25 human volunteers (Kligman 1973). The compound has also been used in the food industry without safety problems for many years and is listed in the Food Chemical Index.

**Table 3**

| **ROUTE** | **SPECIES** | **LDSO** | **REFERENCE** |
|---|---|---|---|
| Oral | Rat | 7800mg/kg | Russell 1973 |
| Oral | Mouse | 4000mg/kg | VPITAR 33(5)48,74 |
| Oral | Guinea Pig | 4000mg/kg | VPITAR 33(5)48,74 |
| Dermal | Rabbit | >5000mg.kg | FRMM |

### Example 4.

A series of *in vitro* tests were carried out to investigate the effects of the compound *trans*-cinnamic acid ethyl ester on the hatching of eggs of *L.sericata.*

A blowfly culture originating from the Central Veterinary Laboratory based in Weybridge, Surrey was used for the tests. None of the original adults had previously been exposed or resistant to insecticides. Eggs aged 10±2 hours of the blowfly were harvested on pig's liver in clusters using a prolene 4/0 paintbrush and examined under the binocular microscope for suitablility. Adult females used to produce these eggs were kept at 20°C, rh 70±5%, with a photoperiod of 8 hours. Groups of 30 eggs were then immediately transferred to a 9-cm Whatman filter paper in a 9-cm plastic petri dish. Two ml of cinnamic acid ethyl ester at concentrations in the range 1.0%; 2.5%; 5.0%; 7.5% and 10.0% V/V was poured gently into the petri dish immersing the eggs. Each concentration was previously prepared by dilution of active compound in a 2.0% W/V aqueous lecithin solution pre-warmed to 20°C. The mixture was sonicated for two minutes. After an exposure time of 1 minute, the test solution was carefully poured off and the eggs recovered individually to a 7ml polythene container in which a filter paper was saturated with pig's liver washings. The L₁ larvae were prevented from escaping by the presence of a 2-cm square piece of cotton cloth, secured in place by the lid of the container which had a 4mm square hole cut out to allow air to enter. Eggs following treatment were incubated at 25°C, rh 70±2% with a photoperiod of 8 hours duration. Hatching typically occurred within 12 hours of treatment.

Each concentration was replicated twice; controls were treated using only lecithin solution. The whole experiment was repeated using two further exposure times of 30 minutes and 90 minutes respectively. Fresh lecithin solution was prepared every day to ensure that bacterial activity did not influence the result.

The data were analysed using fitted curve graphical analysis. LC50 values were calculated from these data. The data gathered from the experiment with an exposure time of 1 minute was also expressed using the probit transformation. However, the remaining data collected from the experiments did not support such analysis due to high egg mortality.

All data was treated according to Abbot W.S. (1925 J. Econ. Ent. 18, 265-267) and expressed as corrected mortality but did not support probit analysis with the exception of exposure time of one minute.

Fitted curves were used to calculate the concentration required to kill 50% of the eggs, (LC50) using the data obtained after immersing the eggs in solutions ranging from 1.0% to 10.0% V/V. The median lethal concentrations, (LC50) and LC95 concentrations are shown in Table 4 below:-

**Table 4**

| **Lethal Concentrations** **at 1 minute** | **Lethal Concentrations** **at 30 minutes** | **Lethal Concentrations** **at 90 minutes** |
|---|---|---|
| LC50 2.76% | LC50 1.78% | LC50 0.36% |
| LC95>7.50% | LC95>5.00% | LC95 2.91% |

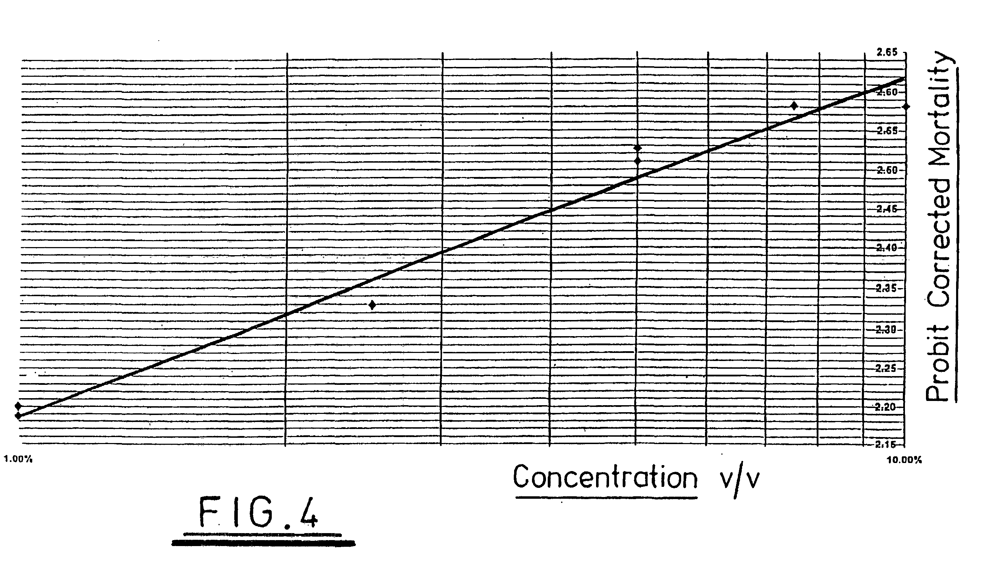

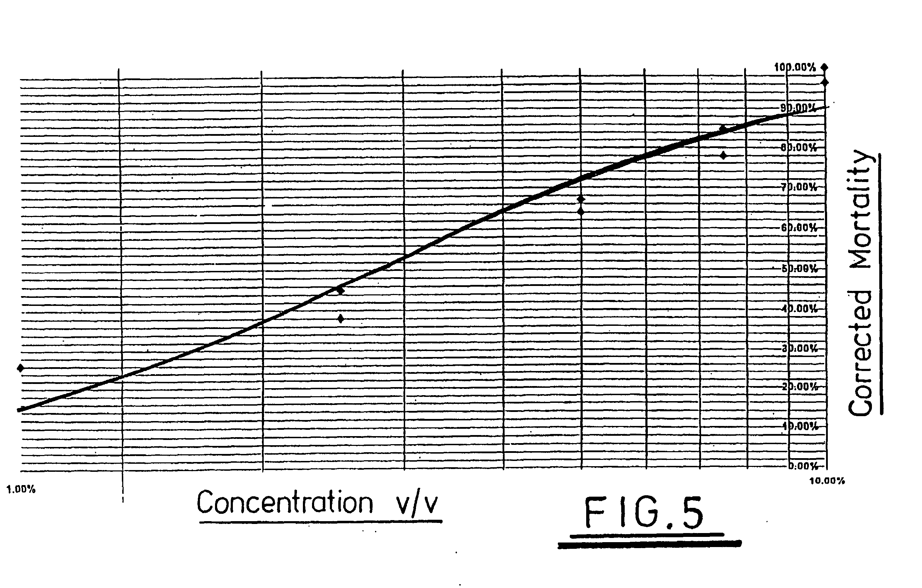

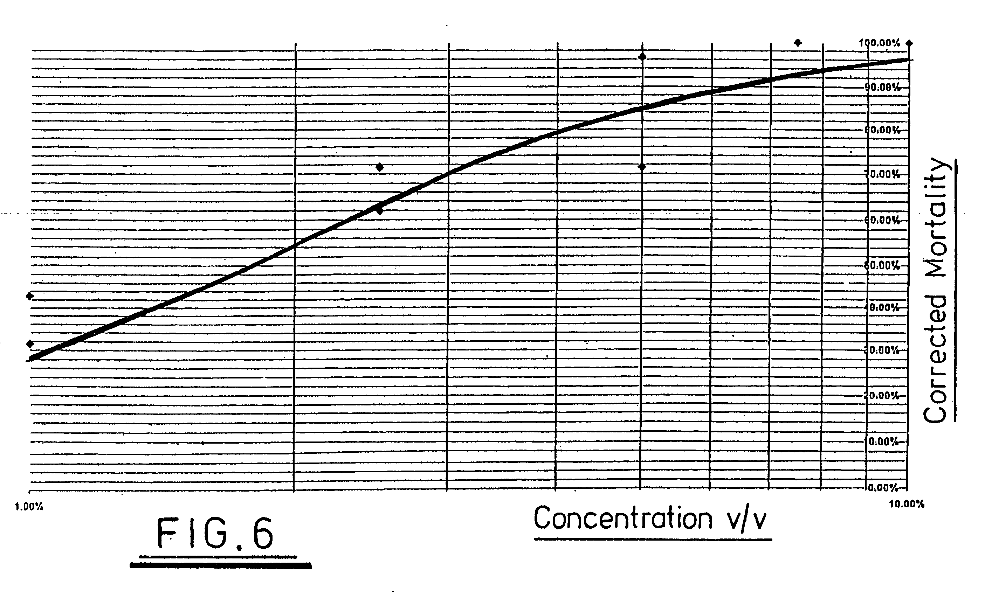

Figures 4, 5 and 6 of the accompanying drawings show efficacy of the compound on the eggs of *L.sericata* after exposure for one minute, thirty minutes and ninety minutes respectively. The graphs show all replicate treatments and demonstrate the trend of increasing mortality as both concentration and exposure time increase. Exposure of the eggs to a concentration of 2.91% V/V provides 95% mortaility at 90 minutes.

The proportion of dead eggs after immersion was analysed using a multiple analysis of variance; (ANOVA) using both concentration and immersion time as factors after arc transforming the corrected mortality data. Both concentration and exposure time are significant, (p=<0.001 in both cases respectively).

Hence, the compound is effective against the eggs of blowflies. However, the agent was found to be ineffective against all stages of blow fly and larval life cycle. It was therefore unexpected that the compound would have an effect on the eggs of the flies. The compound has not previously be used specifically as an ovicide. Its use as an ovicide for the treatment of animals has a major advantage over previous treatments in that it minimizes damage to the skin and tissues of the animals since this is mainly caused by the larvae of the blowfly.

### Example 5

The antifungal activity of ethyl trans-cinnamate against a number of different fungi was investigated by means of in a simple growth inhibition test.

The following quantities of Ethyl trans-Cinnamate were added to Sabourand Dextrose agar (SAB); 50 ml L⁻¹, 4ml L⁻¹, 2ml L⁻¹, 1ml L⁻¹, 400 µl L⁻¹, 200 µl L⁻¹ and 40 µl L⁻¹. The addition of Ethyl trans-Cinnamate did not effect the pH of the Media. These were then autoclaved and poured into standard Petri dishes. Loss of Ethyl trans-Cinnamate due to evaporation during autoclaving was found to be negligible. Duplicate plates of each concentration, plus control plates containing only SAB Agar, where then separately inoculated with, four different species of fungi, these were *Aspergillus* *nidulans, Penicillium digitatum, Rhizopus arrhizus* and *Fusarium culmorum.* Inoculation was carried out using a single stab at the centre of each Petri dish. The plates were then incubated at 30°C for 6 days and daily measurements of mycobial growth where taken every 24 hours. In the case *of Penicillium digitatum,* the presence or absence of growth was recorded, as this species does demonstrate clear radial growth.

The levels of growth for each species, at tested concentrations of Ethyl trans-Cinnamate, are shown in Tables 5a-5d below.

**Table 5a**

| Day | Level of Radial Growth (mm) of *A. nidulans* | | | | | | | |
|---|---|---|---|---|---|---|---|---|
| | 50 ml L⁻¹ | 4ml L⁻¹ | 2ml L⁻¹ | 1ml L⁻¹ | 400µl L⁻¹ | 200µl L⁻¹ | 40µl L⁻¹ | Control |
| 1 | 0 | 0 | 0 | 0 | 0 | 0 | 0 | 2.0 |
| 2 | 0 | 0 | 0 | 0 | 5.5 | 9.5 | 12.0 | 12.5 |
| 3 | 0 | 0 | 0 | 6.0 | 11.5 | 16.5 | 23.0 | 22.5 |
| 4 | 0 | 0 | 0 | 11.5 | 18.0 | 23.0 | 27.0 | 27.0 |
| 5 | 0 | 0 | 6.5 | 16.5 | 24.5 | 28.0 | 32.5 | 32.5 |
| 6 | 0 | 4.5 | 12.5 | 22.0 | 30.0 | 34.5 | 38 | 40.0 |

**Table 5b**

| Day | Level of Radial Growth (mm) of *F. culmorum* | | | | | | | |
|---|---|---|---|---|---|---|---|---|
| | 50 ml L⁻¹ | 4ml L⁻¹ | 2ml L⁻¹ | 1ml L⁻¹ | 400µl L⁻¹ | 200µl L⁻¹ | 40µl L⁻¹ | Control |
| 1 | 0 | 0 | 0 | 0 | 0 | 0 | 0 | 0 |
| 2 | 0 | 0 | 0 | 0 | 0 | 0 | 0 | 0 |
| 3 | 0 | 0 | 0 | 0 | 0 | 0 | 1 | 1 |
| 4 | 0 | 0 | 0 | 0 | 1 | 1 | 1 | 1 |
| 5 | 0 | 0 | 0 | 0 | 2 | 2 | 2 | 2 |
| 6 | 0 | 0 | 0 | 0 | 6 | 6 | 6 | 6 |

**Table 5c**

| Day | Level of Radial Growth (mm) of *R. arrhizus* | | | | | | | |
|---|---|---|---|---|---|---|---|---|
| | 50 ml L⁻¹ | 4ml L⁻¹ | 2ml L⁻¹ | 1ml L⁻¹ | 400µl L⁻¹ | 200µl L⁻¹ | 40µl L⁻¹ | Control |
| 1 | 0 | 0 | 1. | 8.5 | 14.5 | 18.0 | 26.0 | 30.0 |
| 2 | 0 | 23.5 | 45.0 | 45.0 | 45.0 | 45.0 | 45.0 | 45.0 |
| 3 | 0 | 45.0 | 45.0 | 45.0 | 45.0 | 45.0 | 45.0 | 45.0 |
| 4 | 0 | 45.0 | 45.0 | 45.0 | 45.0 | 45.0 | 45.0 | 45.0 |
| 5 | 0 | 45.0 | 45.0 | 45.0 | 45.0 | 45.0 | 45.0 | 45.0 |
| 6 | 0 | 45.0 | 45.0 | 45.0 | 45.0 | 45.0 | 45.0 | 45.0 |
| 45.5mm Growth = Total Plate Coverage. | | | | | | | | |

**Table 5d**

| Day | Growth of *P.* digitatum | | | | | | | |
|---|---|---|---|---|---|---|---|---|
| | 50 ml L⁻¹ | 4ml L⁻¹ | 2ml L⁻¹ | 1ml L⁻¹ | 400µl L⁻¹ | 200µl L⁻¹ | 40µl L⁻¹ | Control |
| 1 | - | - | - | - | - | - | - | + |
| 2 | - | - | - | - | + | ++ | ++ | ++ |
| 3 | - | - | - | - | ++ | ++ | ++ | ++ |
| 4 | - | - | - | + | ++ | ++ | ++ | ++ |
| 5 | - | - | - | + | ++ | ++ | ++ | ++ |
| 6 | - | - | - | + | ++ | ++ | ++ | ++ |
| - = No Growth, + = Growth, ++ = Extensive Growth | | | | | | | | |

It is clear from the results shown in Tables 5a-5d above that Ethyl trans-Cinnamate has an antifungal effect on all four of the different species of fungi tested. At the 50ml L-¹ level there is complete inhibition of growth in all four fungi. Further experiments (data not shown) showed that this effect continued for at least 8 days at this, and higher, doses. Additionally, there is a clearly dose response in the growth of all four fungi. This is particularly clear in *A. nidulans* and the 24h growth of R. *arrhizus,* which show increasing levels of growth.

The data suggests that Ethyl trans-Cinnmnate may be a mycostatic agent, at least at low doses, rather than mycocidal, as all the fungi continue to grow (below 50 ml L-¹) during the experiment, albeit at a slower rate. This is also suggested by the fact that the fast growing *R. arrhizus* covers the plates after only 2 days at the 2ml L-¹ dose, whilst the slower growing *A. nidulans,* and the very slow growing *F*. *culmorum*, never reach this level of growth, the Ethyl trans-Cinnamate having less effect on the faster growing species. Therefore it can be concluded that Ethyl trans-Cinnamate is a very effective antifungal at a dose of 50ml L-¹ and above. This efficacy is reduced as dose is reduced, as would be expected, but a limited effect is still seen at doses as low as as 40µl L⁻¹.

Thus, the compounds of the present invention provide an effective means of controlling parasitic infestations in a number of very different organisms. In particular, the compounds can be used for the combined treatment of infestation, such as scab mite infestation and fly strike, as well as any secondary fungal infection. Moreover, the compounds are an improvement over those compounds previously used for treating infestations because the compounds are non-toxic and environmentally friendly. The compound is particularly useful for inclusion in a sheep dip to prevent/reduce infestation of sheep by Psoroptes or Sarcoptes (mange), especially since the treatment does not impede healing of any lesions present on the sheep nor produce any toxic effects. Additionally, the compound is not harmful to the environment. This is important since many chemicals that have previously been included in sheep dips to treat infestation, such as organophosphate, and pyreithrins are environmentally toxic.

The active compound can be provided in various formulations depending upon its intended purpose and is suitable for use in food, feedstuffs and for animal use due to its low toxicity and low impact on the environment. A concentrated emulsion, e.g. for use as sheep dip, may contain 50% (w/w) *trans-cinnamic* ethyl ester with 47% water and 3% Triton X-100 or powdered (de-oiled) lecithin. Such formulations will typically contain small amounts of an anti-oxidant (such as ascorbic acid or butylated hydroxytoluenc) and a preservative (such as Nipagin, propionic acid or Parabens). The emulsion may be prepared by mixing the various quantities in a high speed blender, such as an Ultra-Turrex homogeniser. Once the high concentration emulsion are obtained, the liquid is then suitable for dilution as appropriate for the application. Solid formulations may also be produced, e.g. for the treatment of stored grains. The active compound is mixed with an insert carrier, such as silica talc, wherein the compound is adsorbed to the particles evenly thereby enabling a well mixed active to be achieved as a dust or powder. Furthermore, the active compound may be included within an oily ointment or in an aqueous cream for topical treatment of domestic animals and/or livestock.

## Claims

1. Use of a compound (I) for the manufacture of a medicament for the treatment of parasitic infestations of livestock, compound (I) having the general formula:- 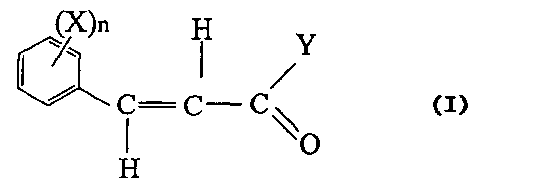 wherein Y is an alkoxy group having 1 to 4 carbon atoms, a hydroxyl group, an amine group, a halide group or a nitro group; X is a hydroxyl group, an amine group, a halide group, a nitro group, an alkoxy group or an ester group and n is 0 or 1

2. Use of the compound (I) for the manufacture of a medicament for the treatment of parasitic infestations selected from the group consisting *of Psoroptes sp., Sarcoptes sp*., *Dermanyssus gallinae* and *Varroa jacobsoni oudemans* (*Varroa destructor),* compound I having the general formula:- 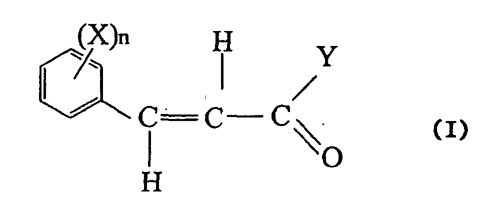 wherein Y is an alkoxy group having 1 to 4 carbon atoms, a hydroxyl group, an amine group, a halide group or a nitro group; X is a hydroxyl group, an amine group, a halide group, a nitro group, an alkoxy group or an ester group and n is 0 or 1.

3. Use of the compound as defined in claim 1 for the treatment of parasitic infestations selected from the group consisting of *Psoroptes sp., Sarcoptes sp., Dermanyssus gallinae* and *Varroa jacobsoni oudemans (Varroa destructor)*

4. Use of the compound as defined in claim 1, 2 or 3 for the combined treatment of *Psoroptes sp.* and *Sarcoptes sp.* infestations in livestock.

5. Use of the compound as defined in claim 1 for the treatment of infestations caused by the eggs of blowflies.

6. Use of the compound as defined in claim 5 for the combined treatment of scab mite infestations and fly strike.

7. Use of the compound as defined in any one of the preceding claims, wherein the compound is trans-cinnamic acid ethyl ester.

8. Use of the compound as defined in any one of the preceding claims wherein the compound is provided as a dilutable emulsion.

9. Use of the compound as defined in claim 8, wherein the emulsifier is sodium lauryl sulphate, Triton-X-100 or lecithin.

10. Use of the compound as defined in claim 9, wherein the emulsifier is included in an amount 1 to 5 wt.%.

11. Use of the compound as defined in claim 10, wherein 3 wt.% of the formulation is emulsifier.

12. Use of the compound as defined in any one of claims 8 to 11, wherein the emulsion is applied as a spray.

13. Use of the compound as defined in any one of claims 8 to 11, wherein the emulsion is applied as a dip.

14. Use of the compound as defined in claim 13, wherein the diluted dip emulsion contains the active compound at a concentration of 0.1 to 10%.

15. Use of the compound as defined in any one of claims 1 to 8, wherein the compound is included with an oily ointment or aqueous cream for topical application.

16. Use of the compound as defined in any one of claims 1 to 8, wherein the compound is to be introduced into an area containing the infested organism by means of a wick based evaporator whereby the compound is vaporized in a sufficient concentration to kill the parasite but does not produce toxic effects in the infested organism.

17. The use of the compound as defined in any one of the preceding claims in combination with other active agents.

18. The use of the compound as defined in claim 17, wherein the other agent is allyl propionate.

## Patentansprüche

1. Verwendung einer Verbindung (I) für die Herstellung eines Medikamentes zur Behandlung von parasitischem Befall eines Tierbestands, wobei die Verbindung (I) die allgemeine Formel aufweist:
wobei Y eine Alkoxygruppe ist, die ein bis vier Kohlenstoffatome aufweist, eine Hydroxylgruppe, eine Aminogruppe, eine Halidgruppe oder eine Nitrogruppe; X ist eine Hydroxylgruppe, eine Aminogruppe, eine Halidgruppe, eine Nitrogruppe, eine Alkoxylgruppe oder eine Estergruppe und n ist 0 oder 1.

2. Verwendung der Verbindung (I) zur Herstellung eines Medikamentes zur Behandlung von parasitischem Befall, wobei die Parasiten aus der Gruppe gewählt wurden, die aus Psoroptes sp., Sarcoptes sp., Dermanyssus gallinae und Varroa jacobsoni oudemans (Varroa destructor) besteht, wobei die Verbindung (I) die allgemeine Formel aufweist:
wobei Y eine Alkoxylgruppe ist, die ein bis vier Kohlenstoffatome aufweist, eine Hydroxylgruppe, eine Aminogruppe, eine Halidgruppe oder eine Nitrogruppe; X ist eine Hydroxylgruppe, eine Aminogruppe, eine Halidgruppe, eine Nitrogruppe, eine Alkoxylgruppe oder eine Estergruppe und n ist 0 oder 1.

3. Verwendung der Verbindung nach Anspruch 1 zur Behandlung von parasitischem Befall, wobei die Parasiten aus der Gruppe gewählt wurden, die aus Psoroptes sp., Sarcoptes sp., Dermanyssus gallinae und Varroa jacobsoni oudemans (Varroa destructor) besteht.

4. Verwendung der Verbindung nach wenigstens einem der Ansprüche 1 bis 3 zur kombinierten Behandlung von Psoroptes sp. und Sarcoptes sp Befall eines Tierbestandes.

5. Verwendung der Verbindung nach Anspruch 1 zur Behandlung von Befall, der durch die Eier von Schmeissfliegen verursacht wurde.

6. Verwendung der Verbindung nach Anspruch 5 zur kombinierten Behandlung von Schorfmilbenbefall und Fliegenbefall.

7. Verwendung der Verbindung nach wenigstens einem der vorhergehenden Ansprüche, wobei die Verbindung ein Transzimtsäureethylester ist.

8. Verwendung der Verbindung nach wenigstens einem der vorhergehenden Ansprüche, wobei die Verbindung als verdünnbare Emulsion bereit gestellt wird.

9. Verwendung der Verbindung nach Anspruch 8, wobei der Emulgator Natriumlaurylsulfat, Triton-X-100 oder Lecithin ist.

10. Verwendung der Verbindung nach Anspruch 9, wobei der Emulgator in einer Menge von 1 bis 5 Gew.% enthalten ist.

11. Verwendung der Verbindung nach Anspruch 10, wobei 3 Gew.% der Mischung ein Emulgator ist.

12. Verwendung der Verbindung nach wenigstens einem der Ansprüche 8 bis 11, wobei die Emulsion als ein Spray angewendet wird.

13. Verwendung der Verbindung nach wenigstens einem der Ansprüche 8 bis 11, **dadurch gekennzeichnet, dass** die Emulsion als ein Bad angewendet wird.

14. Verwendung der Verbindung nach Anspruch 13, wobei die verdünnte Bademulsion die aktive Verbindung in einer Konzentration von 0.1 bis 10% enthält.

15. Verwendung der Verbindung nach wenigstens einem der Ansprüche 1 bis 8, wobei die Verbindung in einer öligen Salbe oder einer wasserhaltigen Creme zur topischen Anwendung enthalten ist.

16. Verwendung der Verbindung nach wenigstens einem der Ansprüche 1 bis 8, wobei die Verbindung auf eine Fläche, die den verseuchten Organismus enthält, durch einen Docht-basierten Verdampfer aufzubringen ist, wobei die Verbindung in einer Menge aufgedampft wird, die ausreichend ist, um den Parasit zu töten, aber keine toxischen Effekte im befallenen Organismus hervorruft.

17. Die Verwendung der Verbindung nach wenigstens einem der vorhergehenden Ansprüche in Kombination mit anderen aktiven Mitteln.

18. Die Verwendung der Verbindung nach Anspruch 17, wobei das andere Mittel Allylproprionat ist.

## Revendications

1. Utilisation d'un composé (I) pour la fabrication d'un médicament pour le traitement des infections parasitaires du bétail, composé (I) ayant la formule générale :
dans laquelle Y est un groupe alcoxy ayant de 1 à 4 atomes de carbone, un groupe hydroxyle, un groupe amine, un groupe halogénure ou un groupe nitro ; X est un groupe hydroxyle, un groupe amine, un groupe halogénure, un groupe nitro, un groupe alcoxy ou un groupe ester et n a la valeur 0 ou 1.

2. Utilisation du composé (I) pour la fabrication d'un médicament pour le traitement des infections parasitaires que l'on choisit parmi le groupe composé de *Psoroptes sp., Sarcoptes sp., Dermanyssus gallinae* et *Varroa jacobsoni oudemans (Varroa destructor),* composé I ayant la formule générale :
dans lequel Y est un groupe alcoxy ayant 1 à 4 atomes de carbone, un groupe hydroxyle, un groupe amine, un groupe halogénure ou un groupe nitro ; X est un groupe hydroxyle, un groupe amine, un groupe halogénure, un groupe nitro, un groupe alcoxy ou un groupe ester et n a la valeur 0 ou 1.

3. Utilisation du composé tel qu'on le définit dans la revendication 1 pour le traitement des infections parasitaires que l'on choisit parmi le groupe composé de *Psoroptes sp., Sarcoptes sp., Dermanyssus gallinae* et *Varroa jacobsoni oudemans (Varroa destructor*).

4. Utilisation du composé tel qu'on le définit dans la revendication 1, 2 ou 3 pour le traitement combiné des infections par *Psoroptes sp.* Et *Sarcoptes sp.* chez le bétail.

5. Utilisation du composé tel qu'on le définit dans la revendication 1 pour le traitement des infections provoquées par les oeufs de mouches à viande.

6. Utilisation du composé tel qu'on le définit dans la revendication 5 pour le traitement combiné des infections par le psoropte commun et la myase (myiase).

7. Utilisation du composé tel qu'on le définit dans l'une quelconque de revendications précédentes, dans laquelle le composé est l'ester éthylique de l'acide *trans*-cinnamique.

8. Utilisation du composé tel qu'on le décrit dans l'une quelconque des revendications précédentes dans laquelle on fournit le composé sous la forme d'une émulsion que l'on peut diluer.

9. Utilisation du composé tel qu'on le définit dans la revendication 8, dans laquelle l'émulsifiant est le lauryl sulfate de sodium, le Triton-X-100 ou la lécithine.

10. Utilisation du composé tel qu'on le définit dans la revendication 9, dans laquelle on inclus l'émulsifiant dans une quantité de 1 à 5% en poids.

11. Utilisation du composé tel qu'on le définit dans la revendication 10, dans laquelle 3% de la formulation en poids est un émulsifiant.

12. Utilisation du composé tel qu'on le définit dans l'une quelconque des revendications 8 à 11, dans laquelle on applique l'émulsion sous la forme d'un aérosol.

13. Utilisation du composé tel qu'on le définit dans l'une quelconque des revendications 8 à 11, dans laquelle on applique l'émulsion sous la forme d'un bain.

14. Utilisation du composé tel qu'on le définit dans la revendication 13, dans laquelle l'émulsion de bain diluée contient le, composé actif à une concentration de 0,1 à 10%.

15. Utilisation du composé tel qu'on le définit dans l'une quelconque des revendications 1 à 8, dans laquelle le composé est inclus avec un onguent huileux ou une crème aqueuse pour une application locale.

16. Utilisation du composé tel qu'on le définit dans l'une quelconque des revendications 1 à 8, dans laquelle on doit introduire le composé dans une zone contenant l'organisme infecté au moyen d'un évaporateur de type à mèche par lequel on vaporise le composé à une concentration suffisante pour tuer le parasite mais sans produire d'effets toxiques dans l'organisme infecté.

17. Utilisation du composé tel qu'on le définit dans l'une quelconque des revendications précédentes en combinaison avec d'autres agents actifs.

18. Utilisation du composé tel qu'on le définit dans la revendication 17, dans laquelle l'autre agent est le propionate d'allyle.
